# EUROPEAN PATENT APPLICATION

(11) **EP 3 163 302 A1**
(43) Date of publication of application: **03.05.2017**
(21) Application number: 15192494.1
(22) Date of filing: 02.11.2015
(51) Int. Cl.: G01N 33/574

(54) **SAMHD1 MODULATION FOR TREATING RESISTANCE TO CANCER THERAPY**

(71) Applicant: Johann Wolfgang Goethe-Universität Frankfurt am Main, 60323 Frankfurt am Main (DE); Rheinische Friedrich-Wilhelms-Universität Bonn, 53012 Bonn (DE)
(72) Inventor: Keppler, Oliver Till, 80638 München (DE); Cinatl, Jindrich, 63069 Offenbach / Main (DE); Schneider, Constanze, 60599 Frankfurt am Main (DE); Baldauf, Hanna-Mari, 77815 Bühl (DE); Schwarz, Sarah-Marie, 60599 Frankfurt/Main (DE); Serve, Hubert, 60431 Frankfurt (DE); Oellerich, Thomas, Cambridge, CB2 7EB (GB); Geisslinger, Gerd, 65812 Bad Soden (DE); Hornung, Veit, 81245 München (DE)
(74) Representative: Krauss, Jan

(57) **Abstract**

The present invention pertains to novel treatments for cancer diseases. Treatment of cancers with nucleoside analogs (NA), which specifically inhibit rapidly dividing cells, may face preexisting NA resistance or development of resistant cancer cells resulting in poor clinical prognosis. Treatment of cancers with oncolytic herpes simplex viruses (HSV) may face preexisting resistance or development of resistant cancer cells resulting in poor clinical prognosis. The invention overcomes chemotherapy resistance or resistance to oncolytic HSV by providing methods for detecting the resistance in a cancer disease based on the expression of SAM domain and HD domain-containing protein 1 (SAMHD1) in cancer cells. Furthermore provided are treatment options addressing the chemotherapy resistance such as a combination of a SAMHD1 inhibitor with a NA. Moreover, provided are treatment options addressing the resistance to oncolytic HSV such as a combination of a SAMHD1 inhibitor or depletion of SAMHD1 with an oncolytic HSV. The invention provides new medicines and companion diagnostics supporting clinical treatment decisions.

## Description

### FIELD OF THE INVENTION

The present invention pertains to novel treatments for cancer diseases. Treatment of cancers with nucleoside analogs (NA), which specifically inhibit rapidly dividing cells, may face preexisting NA resistance or development of resistant cancer cells resulting in poor clinical prognosis. Treatment of cancers with oncolytic herpes simplex viruses (HSV) may face preexisting resistance or development of resistant cancer cells resulting in poor clinical prognosis. The invention overcomes chemotherapy resistance or resistance to oncolytic HSV by providing methods for detecting the resistance in a cancer disease based on the expression of SAM domain and HD domain-containing protein 1 (SAMHD1) in cancer cells. Furthermore provided are treatment options addressing the chemotherapy resistance such as a combination of a SAMHD1 inhibitor with a NA. Moreover, provided are treatment options addressing the resistance to oncolytic HSV such as a combination of a SAMHD1 inhibitor or depletion of SAMHD1 with an oncolytic HSV. The invention provides new medicines and companion diagnostics supporting clinical treatment decisions.

### DESCRIPTION

The use of chemical drugs as cytostatics for cancer therapy constitutes one of the choices as first-line treatment for the majority of solid and hematopoietic tumors. The most commonly used chemical drugs for cancer therapy are: platin agents, topoisomerase inhibitors, vinca alkaloids, taxanes and nucleoside analogs (NA) that tackle specifically rapidly dividing cells, however, via different mechanisms. Often first line treatments are insufficient to destroy all tumor cells from the organism or have significant adverse effects. Also drug resistance is recognized as the primary cause of failure of cancer therapy when chemotherapeutic agents are applied. In spite of that, sub-optimal drug concentration on the tumor milieu could influence the drug resistance; other factors like cellular origin plays an essential role for chemo-resistance of many tumors. Drug resistance is a multifactorial phenomenon depending on multiple independent mechanisms which involve intracellular detoxification, changes of the cellular response, tolerance to stress and defects in apoptosis signaling pathways. The glycoprotein-P and the gluthathion S-transferase are the major proteins that mediate the intracellular detoxification process linked to well established modes of drug resistance in cancer. Other proteins like beta-tubulins have been reported to be involved in the drug resistance phenomenon and whose levels directly correlate with the tumor resistance to Paclitaxel. Moreover, the cisplatin resistance has been reported to be influenced by the over-expression of different proteins like T-plastin, the heat shock protein (HSP70) and (HSP90) and the transcription factor YB1. Reports from different groups have indicated the existence of a set of proteins which either inhibit apoptosis or increase cell survival on tumor cells thus contributing to the chemo-resistance phenomenon of tumors.

NAs are therapeutically inactive molecules, which have to be activated in tumor cells through different cellular enzymes into the corresponding potent nucleoside triphosphate (NTP) and deoxy-nucleoside triphosphate (dNTP) analogs. Consequently, resistance of tumor cells to NAs may be associated with changes in expression or gene mutations of the enzymes involved in the activation of NAs to NTP analogs.

There are considerable efforts to increase therapeutic efficacy of nucleoside analogs used for treatment of leukemia or glioma patients especially those with recurrent disease. Clinically tested approaches involve combinations of NAs with one or more other NAs or with inhibitors of signaling pathways relevant for leukemogenesis. These treatment strategies combine drugs which exert cytotoxic effects and mostly influence more than one target in human cells. Consequently, unprecedented toxicity in treated patients has frequently occurred and synergistic activities are poorly defined.

SAM domain and HD domain-containing protein 1 (SAMHD1) is a cellular enzyme activated by GTP or dGTP and which has a triphosphohydrolase activity (dNTPase). The enzyme catalyzes the hydrolysis of dNTPs into component nucleosides and an inorganic triphosphate. Therefore the enzyme is involved in the depletion of cellular dNTP pools and counteracts cell division processes. By reducing the dNTP substrate levels required for reverse transcription of the HIV RNA into a complementary DNA, the enzyme is also responsible for blocking replication of HIV in dendritic cells, macrophages, monocytes, and resting CD4 T-cells. Different experimental tools and naturally evolved mechanisms exist to deplete SAMHD1 from cells including siRNA/shRNA, CRISP/CAS9, as well as Vpx proteins from HIV-2 and SIV and Vpr proteins from SIV (Baldauf, 2012). Methods for quantitation of endogenous levels of NTPs and dNTPs in human cells, e.g. by liquid chromatography tandem mass spectrometry, were established (Thomas, 2015). Enzymatic assays for the triphosphohydrolase activity of SAMHD1 to determine the mode of interaction of NTPAs and NTPAs with SAMHD1 were also developed (Arnold 2014). A number of monoclonal and polyclonal antibodies for quantitative detection of SAMHD1 in leukemic and other cells are commercially available. Technologies for targeting of leukemic cells and other cancer cells using delivery systems and/or coupling to antibodies specific for leukemia or the respective cancer cell have been described and tested in clinical trials (Row 2013).

In view of the state of the art the present invention provides a novel approach for overcoming chemotherapy resistance that naturally exists or develops in response to a first line treatment with NAs, in particular in the context of a treatment of leukemia or glioma, but is not restricted to these types of cancer.

The above problem is solved in a first aspect by a method for determining resistance of a cancer patient to a cancer therapy, comprising the steps of
a. Determining the level of SAM domain and HD domain-containing protein 1 (SAMHD1) in a sample from the cancer disease of the cancer patient,
b. Comparing the expression as determined in (a) with a control, or reference,
wherein the presence of, or higher level of, SAMHD1 in the sample compared to the control or reference indicates resistance of the cancer patient to the cancer therapy, or wherein the absence of, or lower level of, SAMHD1 in the sample compared to the control or reference indicates susceptibility of the cancer patient to the cancer therapy.

In another aspect the problem of the invention is solved by an *in-vitro* method for stratifying a cancer patient as responder or non-responder to a cancer therapy, comprising the steps of
a. Determining the level of SAMHD1 in a sample from the cancer disease of the cancer patient,
b. Comparing the expression as determined in (a) with a control or reference,
wherein the presence of, or higher level of, SAMHD1 in the sample compared to the control or reference indicates the cancer patient as non-responder to the cancer therapy, or wherein the absence of, or lower level of, SAMHD1 in the sample compared to the control or reference indicates the cancer patient as responder to the cancer therapy.

A "different level" in context of the invention shall refer to either an increased (higher) or decreased (lower) level compared to a control or reference. For example in some embodiments an increased level may be indicative for the presence of a chemotherapy resistance or resistance against an oncolytic virus. Then a decreased or equal level would be indicative for the absence of the chemotherapy resistance or resistance against an oncolytic virus. In other embodiments an equal or increased level may be indicative for the presence of a chemotherapy resistance or resistance against an oncolytic virus. Then a decrease of the level would be indicative for the absence of the chemotherapy resistance or resistance against an oncolytic virus.

The term "sample" or "biological sample" means any biological sample derived from a subject or patient. Examples of such samples include tissues, cell samples, cell lysates, biopsies, etc. Biological samples may be selected from a tumor sample or a biopsy such as a sample from a solid glioma. Other biological samples are whole blood, serum or plasma. Preferably, the sample is a whole blood sample. Most preferred in context of the present invention is that the biological sample is a blood sample, a bone marrow sample, or a biopsy sample. The nature of the sample will depend on the cancer disease diagnosed or treated in context with the herein described invention.

Preferred for the present invention is that the sample of the cancer disease is a sample containing cancer cells of the cancer disease, for example from a resected tumor.

The term "determining" as used herein includes qualitative and/or quantitative detection (i.e. detecting and/or measuring expression level) with or without reference to a control or a predetermined value. "Determining the level" shall refer to a quantitative detection of a biomarker as disclosed herein. For determining the level of SAMHD1 in the biological sample and/or control sample any method can be used that allows the quantification of SAMHD1 concentrations. In a preferred embodiment the content of SAMHD1 in a sample to be analyzed is determined immunologically by using a SAMHD1-specific antibody or mass spectrometry (protein detection) or by quantitative real time-PCR (qPCR) (mRNA detection). In particular "determining the level of SAMHD1" in context of the present invention may include both direct determination of SAMHD1 protein or mRNA expression, as well as indirect determination of the level of SAMHD1 as for example detection of negative or positive regulators of SAMHD1 expression and/or function. Such a negative regulator of SAMHD1 may be an inhibitory nucleic acid, such as a microRNA, for example miRNA 181a or 181b. Cytokines or other bioactives may function as positive or negative regulators of SAMHD1 expression and/or function.

The term "subject," as used herein, describes a mammal including, but not limited to, humans, apes, chimpanzees, orangutans, monkeys, dogs, cats, horses, pigs, sheep, goats, mice, rats, and guinea pigs. However, in some embodiments a subject is preferably a human. In certain embodiments, where the methods of the present invention are used for determining the presence of a chemotherapy resistance, the subject is a subject suffering from cancer disease, such as a recurrent cancer disease.

The term "level" in conjunction with one or more biomarkers of the present disclosure shall refer preferably to the concentration of the respective biomarker.

The term "control" as used in the context of the present invention may refer to various reference values depending on the diagnostic context for which the present method is used. A control level may therefore be any reference value of the respective biomarker which allows for a meaningful interpretation of the status or development of a chemotherapy resistance or resistance against an oncolytic virus in a patient having a cancer disease. In general, a control level may correspond to a level of the biomarker in cancer cells having different levels of SAMHD1 expression (high, medium, low) not having or displaying the malignancy. Alternatively, a control level may correspond to a level of SAMHD1 in a biological sample of the subject at an earlier time point, for example, before said subject underwent chemotherapy or other medical treatments. In certain embodiments of the invention the control level is a cut-off level, and an increased level or equal level of SAMHD1 in the biological sample compared to the cut-off level is indicative for the presence of the chemotherapy resistance or resistance against an oncolytic virus. Most preferably, an increased level or equal level of SAMHD1 in the biological sample compared to the cut-off level is indicative for the presence of chemotherapy resistance or resistance against an oncolytic virus in the subject.

In certain embodiments of the invention the cancer therapy is a chemotherapy, such as a therapy with a nucleoside analog (NA), or a therapy with an oncolytic virus, such as an oncolytic Herpes simplex virus (HSV).

The term "cancer disease" disclosed herein comprises lung cancer, arseniccellular lung cancer, liver cancer, colon cancer, bone cancer, pancreatic cancer, skin cancer, cephalic or cervical cancer, skin or endophthalmic melanoma, hysterocarcinoma, ovarian cancer, rectal cancer, stomach cancer, perianal cancer, colonic cancer, breast cancer, endometrioma, cervical carcinoma, vaginal carcinoma, vulval carcinoma, Hodgkin's disease, esophageal cancer, enteric cancer, endocrine gland cancer, thyroid cancer, parathyroid cancer, adrenal cancer, smooth tissue sarcoma, urethral cancer, penile cancer, prostatic cancer, chronic or acute leukemia, lymphocytoma, cystic cancer, nephritic or hydrouretic cancer, renal cell carcinoma, renal pelvic carcinoma, CNS tumor, primary CNS lymphoma, spinal medulla tumor, brain stem neuroglioma, hypophyseal adenomatosis and the like, preferably, lung cancer, liver cancer, skin cancer or endophthalmic melanoma. Preferred cancers are glioma, glioblastoma, or a leukemia, such as AML, ALL, CLL, or CML, and/or wherein the cancer disease is a recurrent cancer disease.

In context of the present invention a chemotherapy or a chemotherapeutic is an NA, for example selected from didanosine, vidarabine, BCX4430, cytarabine, emtricitabine, lamivudine, zalcitabine, abacavir, aciclovir, entecavir, stavudine, telbiv-udine, zidovudine, idoxuridine, nelarabine, fludarabin, or trifluridine. The most preferred NA is cytarabine.

The term "oncolytic virus" is meant to comprise any virus that infects/enters and lyses cancer cells. The ideal oncolytic virus efficiently kills a clinically relevant fraction of the patient's cancer cells by direct cytolysis with a minimal destruction of non-neoplastic tissue. Targeted tumor cell entry and specificity of replication are desirable. Furthermore, the virus should be safe and apathogenic when applied in patients. Oncolytic viruses derived from many different types of viruses have been described by Liu et al. (Liu et al., Nature Clinical Practice Oncology 4: (2) 101-117, 2007). Among these enveloped viruses such as herpes simplex virus (HSV), vaccinia virus (VV) and paramyxoviruses such as measles virus (MeV), Newcastle disease virus (NDV) or rhabdoviruses like vesicular stomatitis virus (VSV), are most prominent. Besides applying unmodified wildtype virus, genetic engineering can further improve safety and efficacy of oncolytic viruses. Engineering the envelope proteins can restrict virus infection to tumor cells and insertion of suicide genes can enhance therapeutic effects (Nakamura et al., Expert Opin. Bio. Ther. 4: (10): 1685-1692, 2004); Liu et al., Nature Clinical Practice Oncology 4: (2) 101-117, 2007). Most preferred is a HSV virus.

Yet another aspect of the invention provides a method of selecting a cancer therapy for treating a cancer patient, comprising the steps of
a. Determining the level of SAMHD1 in a sample from the cancer disease of the cancer patient,
b. Comparing the expression as determined in (a) with a control or reference,
wherein the absence of, or lower level of, SAMHD1 in the sample compared to the control or reference indicates a chemotherapeutic cancer therapy or a cancer therapy with an oncolytic virus for treating the cancer patient, and wherein the presence of, or a higher level of, SAMHD1 in the sample compared to the control or reference indicates a combination cancer therapy comprising a SAMHD1 inhibitor with a chemotherapeutic or oncolytic virus.

Another aspect also relates to a method for monitoring a cancer therapy of a cancer disease, wherein during the cancer therapy, chemotherapy resistance of the cancer disease is determined according to any of the aforementioned methods for detecting resistance to cancer therapy or for stratifying patients.

It is in some embodiments preferably that chemotherapy resistance of the cancer disease is determined at least twice during the chemotherapy, more preferably regularly during the therapy, e.g. monthly.

All of the methods disclosed herein may comprise an additional first step of providing a biological sample from the subject/patient.

Any of the herein above or below described methods are in preferred embodiments methods excluding any steps performed at the human or animal body. In some preferred embodiments the herein disclosed methods are strictly performed *in vitro* or *ex vivo.* In particular the term "providing a biological sample from the subject" should not be misinterpreted to explicitly or implicitly include an invasive method step of obtaining the biological sample from a living subject (animal or human). The term "providing a biological sample" shall explicitly exclude any steps of obtaining a biological sample from a subject.

Another aspect of the invention then pertains to an NA or oncolytic virus for use in the treatment of a cancer disease in a subject, wherein the treatment comprises, obtaining from the subject a sample from the cancer disease, and determining chemotherapy resistance of the cancer disease in the subject according to the method described herein above using the sample from the cancer dis-ease, wherein if a chemotherapy resistance is indicated for the cancer disease, the treatment is terminated, and wherein if no chemotherapy-resistance is indicated, the treatment is continued.

If a chemotherapy resistance is indicated for the cancer disease, the treatment may be terminated and a second treatment is commenced comprising the administration of a combination of a NA and a SAMHD1 inhibitor.

Another aspect of the invention then pertains to a SAMHD1 inhibitor or antagonist for use in the treatment of a cancer disease, wherein the treatment comprises the concomitant or sequential administration of an SAMHD1 inhibitor and a second therapeutic selected from an NA or oncolytic virus.

As used herein, the terms "SAMHD1-antagonist" or "SAMHD1-inhibitor" are used synonymously. The term refers to a substance that affects a decrease in the amount or rate of SAMHD1 expression or activity. Such a substance can act directly, for example, by binding to SAMHD1 and decreasing the amount or rate of SAMHD1 expression or activity. A SAMHD1-antagonist can also decrease the amount or rate of SAMHD1 expression or activity, for example, by binding to SAMHD1 in such a way as to reduce or prevent SAMHD1 enzymatic activity; by binding to SAMHD1 and modifying it, such as by removal or addition of a moiety; and by binding to SAMHD1 and reducing its stability. A SAMHD1-antagonist can also act indirectly, for example, by binding to a regulatory molecule in the cell or at the cell surface, such as a cytokine receptor, or a gene region so as to modulate regulatory protein or gene region function and affect a decrease in the amount or rate of SAMHD1 expression or activity. Such activities may transiently or permanently affect SAMHD1 expression or function in a cancer cell. Thus, a SAMHD1-antagonist can act by any mechanisms that result in decrease in the amount or rate of SAMHD1 expression or activity.

A SAMHD1-antagonist can be, for example, a naturally or non-naturally occurring macromolecule, such as a polypeptide, peptide, peptidomimetic, nucleic acid, carbohydrate or lipid. A SAMHD1-antagonist further can be an antibody, or antigen-binding fragment thereof, such as a mono-clonal antibody, humanized antibody, chimeric antibody, minibody, bifunctional antibody, single chain antibody (scFv), variable region fragment (Fv or Fd), Fab or F(ab)2. A SAMHD1-antagonist can also be polyclonal antibodies specific for SAMHD1. A SAMHD1-antagonist further can be a partially or completely synthetic derivative, analog or mimetic of a naturally occurring macromolecule, or a small organic or inorganic molecule.

A SAMHD1-antagonist that is an antibody can be, for example, an antibody that binds to SAMHD1 and inhibits its catalytic center, or alters the activity of a molecule that regulates SAMHD1 expression or activity, such that the amount or rate of SAMHD1 expression or activity is decreased. An antibody useful in a method of the invention can be a naturally occurring antibody, including a monoclonal or polyclonal antibody or fragment thereof, or a non-naturally occurring antibody, including but not limited to a single chain antibody, chimeric antibody, bifunctional antibody, complementarity determining region-grafted (CDR-grafted) antibody and humanized antibody or an antigen-binding fragment thereof. In addition to antibodies the present invention may also relate to SAMHD1-specific and -inhibitory T-cell receptors.

A SAMHD1-antagonist that is a nucleic acid can be, for example, an anti-sense nucleotide sequence, an RNA molecule, or an aptamer sequence. An anti-sense nucleotide sequence can bind to a nucleotide sequence within a cell and modulate the level of expression of SAMHD1, or modulate expression of another gene that controls the expression or activity of SAMHD1. Similarly, an RNA molecule, such as a catalytic ribozyme, can bind to and alter the expression of the SAMHD1 gene, or other gene that controls the expression or activity of SAMHD1. An aptamer is a nucleic acid sequence that has a three dimensional structure capable of binding to a molecular target. Specifically preferred in context of the present invention are expression constructs of miR181a or 181b, which were shown to be a negative regulator for SAMHD1 expression. Also included are vector constructs for targeted knock out or mutation of SAMHD1 for example by the CRISPR-Cas9 technology or similar targeted gene editing approaches.

A SAMHD1-antagonist that is a nucleic acid also can be a double-stranded RNA molecule for use in RNA interference methods. RNA interference (RNAi) is a process of sequence-specific gene silencing by post-transcriptional RNA degradation, which is initiated by double-stranded RNA (dsRNA) homologous in sequence to the silenced gene. A suitable double-stranded RNA (dsRNA) for RNAi contains sense and antisense strands of about 21 contiguous nucleotides corresponding to the gene to be targeted that form 19 RNA base pairs, leaving overhangs of two nucleotides at each 3' end (Elbashir et al., Nature 411:494-498 (2001); Bass, Nature 411:428-429 (2001); Zamore, Nat. Struct. Biol. 8:746-750 (2001)). dsRNAs of about 25-30 nucleotides have also been used successfully for RNAi (Karabinos et al., Proc. Natl. Acad. Sci. USA 98:7863-7868 (2001)). dsRNA can be synthesized in vitro and introduced into a cell by methods known in the art.

Other preferred embodiments pertain to SAMHD1 inhibitory viral proteins. In this case proteins such as lentiviral protein X (Vpx) or viral protein R (Vpr) are preferred examples. For instance the accessory protein Vpx is encoded by HIV-2, SIV_{SM}, or SIV_{MAC}, and/or wherein the accessory protein Vpr is encoded by SIVₘᵤₛ and SIV_{deb}.

In certain embodiments the treatment aspect described by the present invention relates to the treatment of a cancer disease which is a solid cancer disease, such as glioma, or leukemia, such as AML, and/or a recurrent cancer disease.

Another aspect of the invention relates to a combination of a SAMHD1 inhibitor and a second therapeutic agent selected from an oncolytic virus, for use in the treatment of a cancer disease.

In this context, the term "combination" refers to an active substance combination of two or more active substances in a formulation and also as a combination in the sense of individual formulations of the active substances administered at specified intervals from one another in a therapeutic treatment. Thus the term "combination" shall include the clinical reality of a co-administration of two therapeutically effective compounds, as is described in context of the present invention.

Co-administration: In the context of the present application, co-administration of two compounds is defined as administration of the two compounds to the patient within one year, including separate administration of two medicaments each containing one of the compounds as well as simultaneous administration whether or not the two compounds are combined in one formulation or whether they are in two separate formulations.

The combination of the invention in one embodiment includes that the SAMHD1 inhibitor and the second therapeutic agent are combined by sequential or concomitant administration to a subject during said prevention or treatment, preferably wherein the antagonists and chemo-therapeutics are concomitantly administered during said prevention or treatment.

The second therapeutic agent is preferably selected from an oncolytic virus and NA as described herein before.

Another aspect of the invention then pertains to a diagnostic kit, comprising means for determining the presence or absence of SAMHD1 in a sample from a cancer disease. Such means for determining the presence, or absence, or level, of SAMHD1 may be selected from polynucleotide probes and/or primer, or antibodies.

Nonwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

The present invention will now be further described in the following examples with reference to the accompanying figures and sequences, nevertheless, without being limited thereto. For the purposes of the present invention, all references as cited herein are incorporated by reference in their entireties. In the Figures:

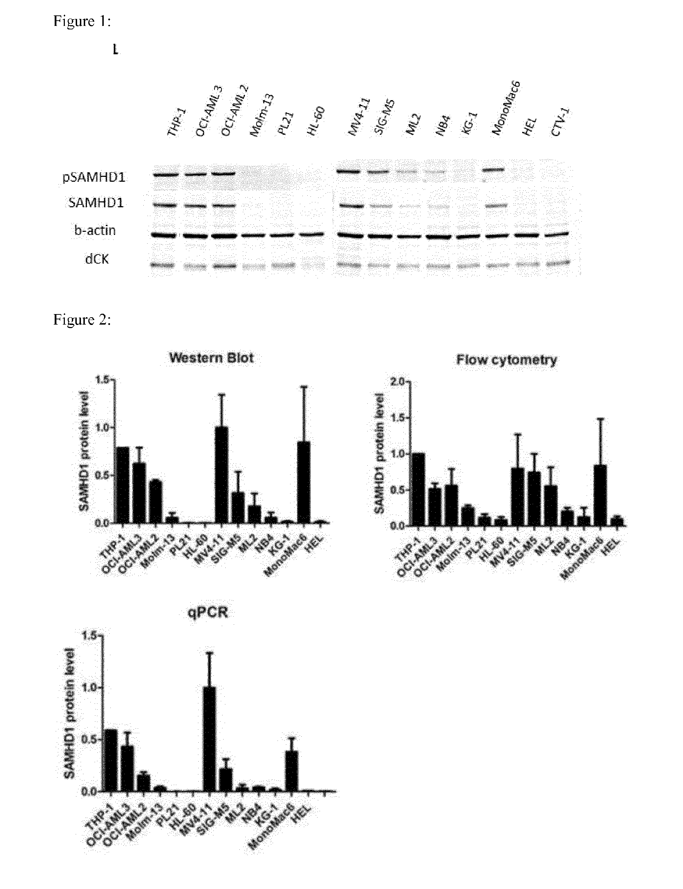

- **Figure 1:**: Western Blot analysis of SAMHD1, pSAMHD1 (T592), dCK and β-actin as loading control in AML cell lines.
- **Figure 2:**: Quantification of expression levels of SAMHD1 protein and mRNA in different leukemic cell lines. Error bars are means +/- standard deviations.

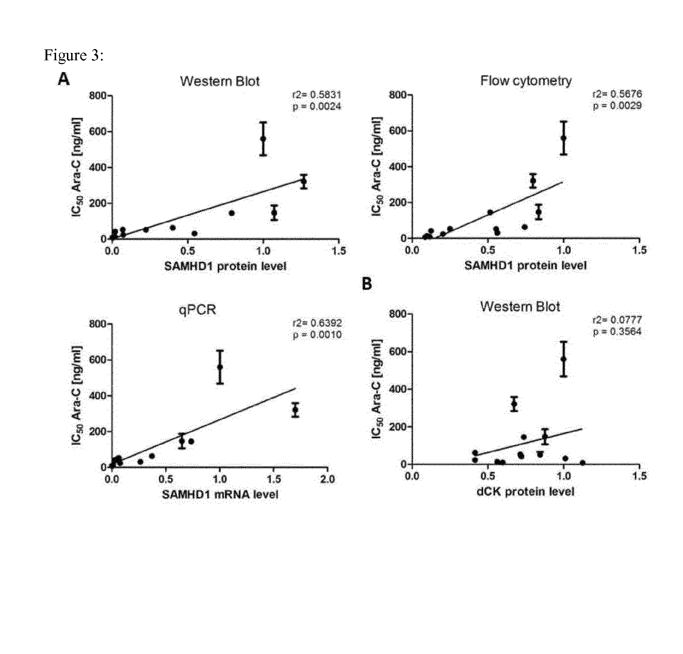

- **Figure 3:**: Spearman correlation between Ara-C concentrations inhibiting 50 % cell growth (IC₅₀) and the expression levels of SAMHD1 (**A**) and between IC₅₀ and the expression levels of dCK **(B).**

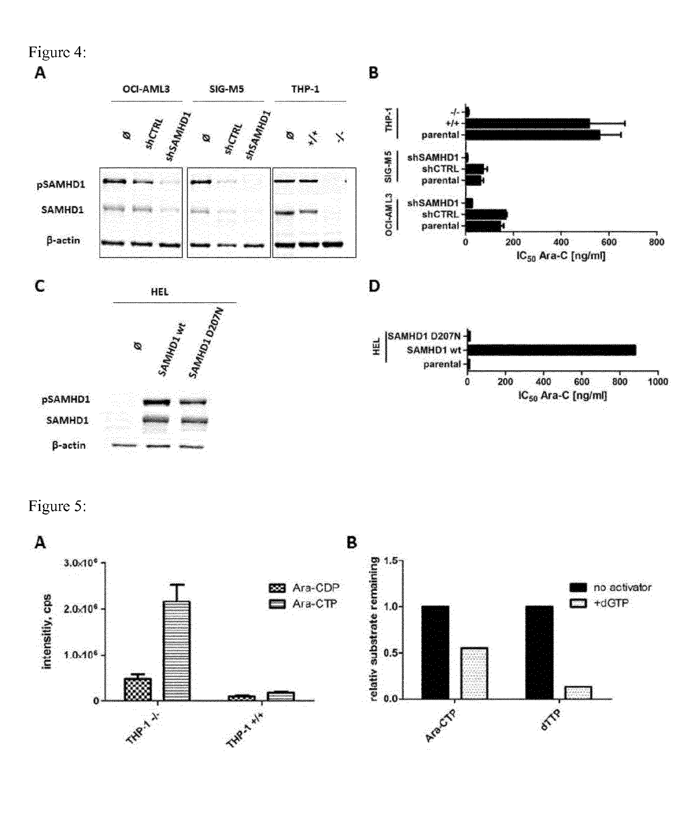

- **Figure 4:**: Expression levels of SAMHD1 and pSAMHD1 (**A**) and Ara-C concentrations inhibiting 50 % cell growth (IC₅₀) **(B)** in leukemic cell lines with depleted SAMHD1 using shRNA or CRISP/Cas9 technology. Expression levels of wtSAMHD1 and the SAMHD1 D207N mutant **(C)** and IC₅₀ values of Ara-C **(D)** in parental HEL and HEL cells expressing wtSAMHD1 or the SAMHD1 D207N mutant.
- **Figure 5:**: Formation of pharmacologically active metabolite of cytarabine, Ara-CTP, and Ara-CDP in CRISP/CAS9 SAMHD1 knock-out THP-1 (-/-) cells or control THP-1 (+/+) cells expressing wt SAMHD1 **(A).** Ara-CTP and dTTP hydrolysis by SAMHD1 evaluated in enzymatic endpoint assay. dGTP was used as activator of SAMHD1 (**B**).

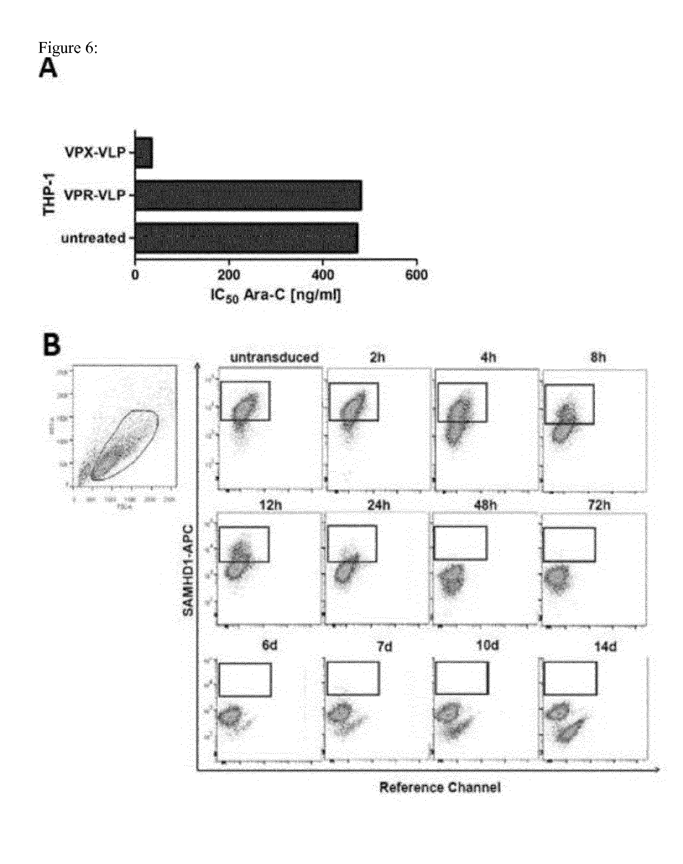

- **Figure 6:**: Cytotoxic effects of Ara-C in THP-1 cells treated HIV-2/Vpx or non-degrading HIV-1/Vpr VLPs (Vpx-VLPs and or Vpx-VLPs, respectively) or untreated cells (**A**). Effects of Vpx-VLPs or Vpr-VLPs on SAMHD1 levels in THP-1 cells measured by flow cytometry **(B).** For **A**, error bars are mean +/- standard deviations.

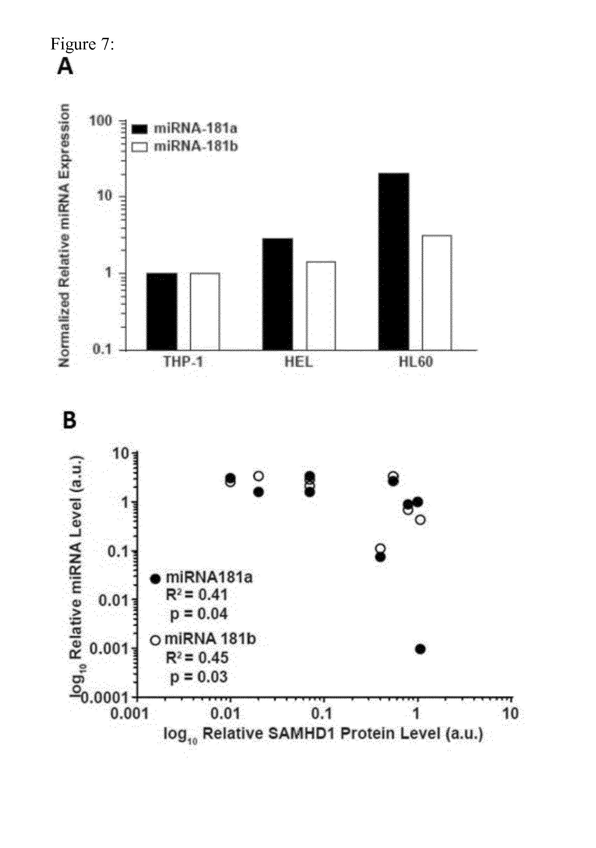

- **Figure 7:**: miRNA181a and miRNA181b expression levels in different leukemic cell lines (**A**). Spearman correlation between expression levels of miRNA181a/miRNA181b and SAMHD1 protein **(B).**

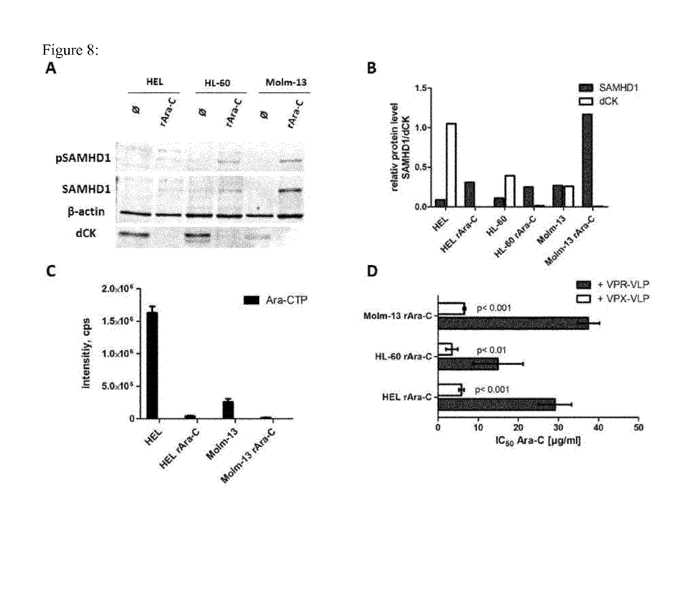

- **Figure 8:**: Expression levels of SAMHD1, pSAMHD1, and dCK protein determined by Western Blot (**A** and **B).** Ara-CTP and Ara-CDP intensities evaluated by liquid chromatography tandem mass spectrometry **(C)** and Ara-C concentrations inhibiting 50 % cell growth (IC₅₀) in parental HEL, HL-60, Molm-13 and Ara-C resistant sublines HELrAra-C, HL-60rAra-C and Molm-13rAra-C.

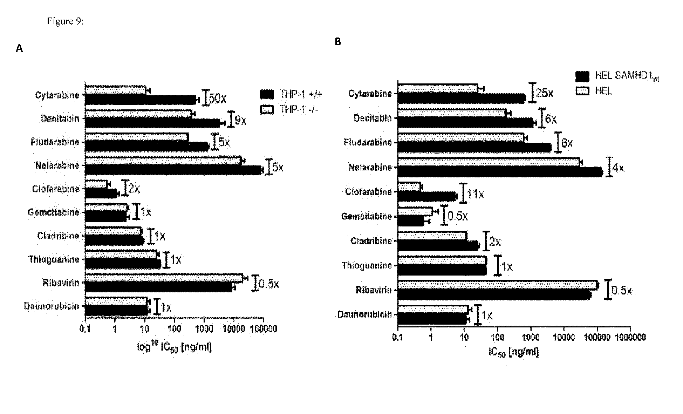

- **Figure 9:**: Concentrations inhibiting 50 % cell growth (IC₅₀) of clinically relevant NAs and daunorubicin of CRISP/CAS9 SAMHD1 knock-out THP-1 (-/-) cells or control THP-1 (+/+) cells expressing wt SAMHD1 (**A**) and in parental HEL cells (SAMHD1 low) and HEL cells engineered to constitutively express wt SAMHD1 **(B).** Error bars are mean +/- standard deviations.

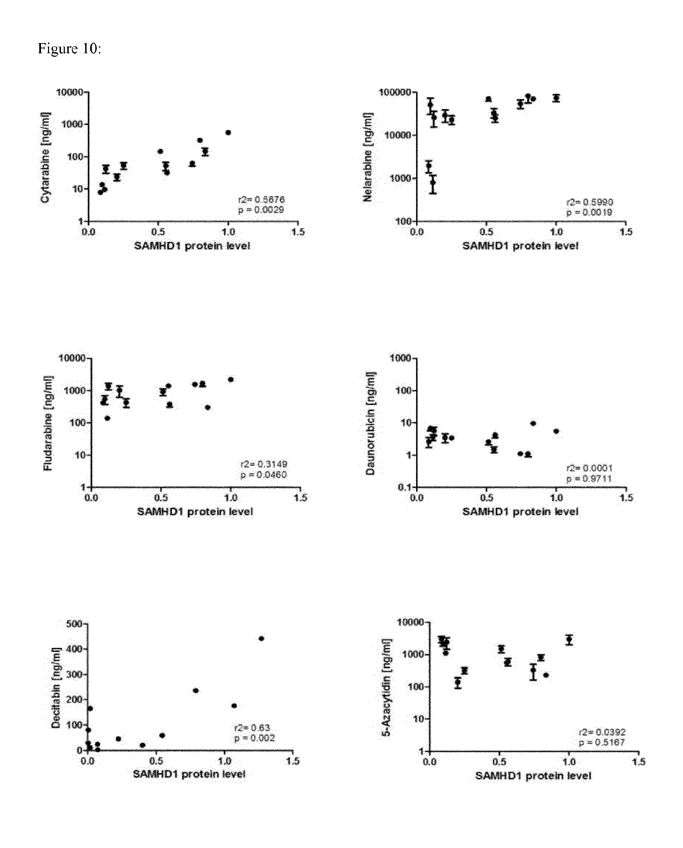

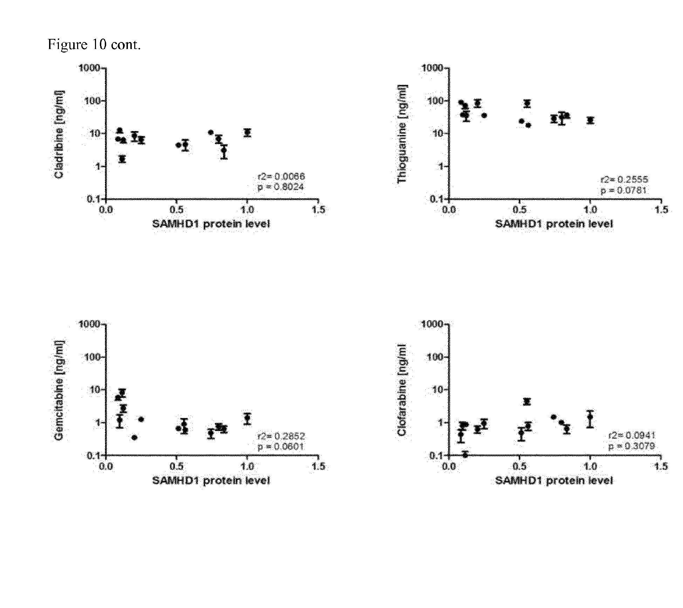

- **Figure 10:**: Spearman correlation between concentrations inhibiting 50 % cell growth (IC₅₀) of clinically relevant drugs (including nucleoside analogues and daunorubicin) and SAMHD1 expression levels in 13 AML cell lines.

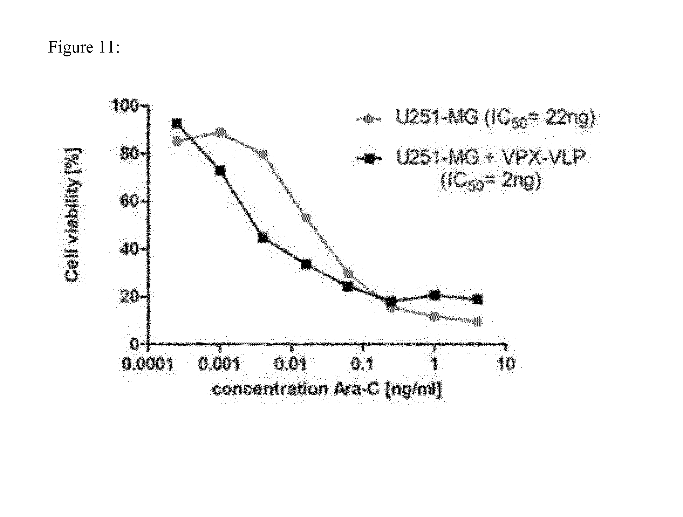

- **Figure 11:**: Effect of Vpx-VLPs on cytotoxic activity of cytarabine in glioma cell line U251-MG.

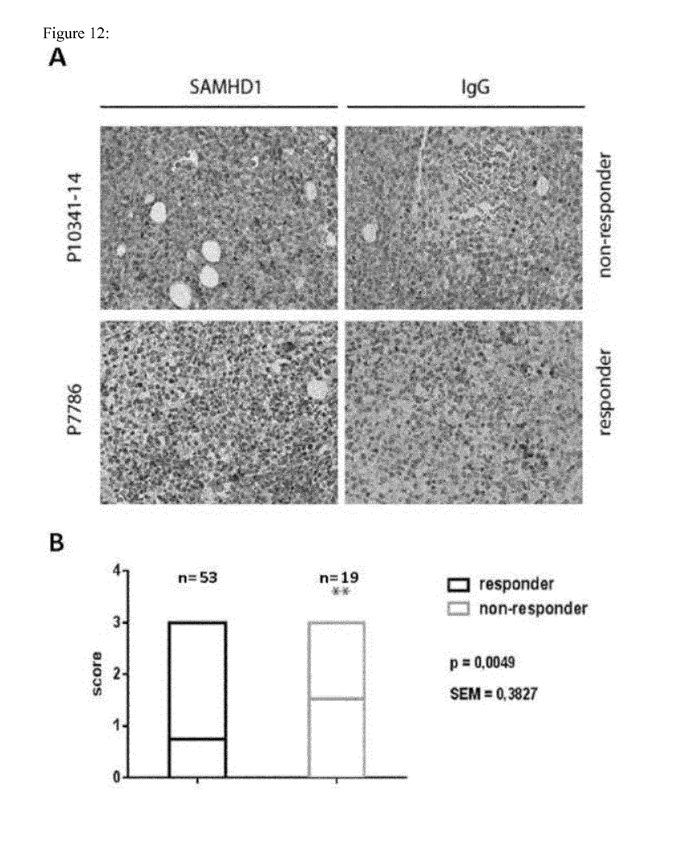

- **Figure 12:**: Bone marrow biopsies for a patient who responded to cytarabine therapy ("responder") and for a patient who did not respond to cytarabine therapy ("non-responder") (**A**). Expression levels of SAMHD1 in bone marrow biopsies of 19 patients who did not respond and 53 patients who exerted response to cytarabine therapy **(B).** Statistical significance was assessed using paired Student'*t-*test.s

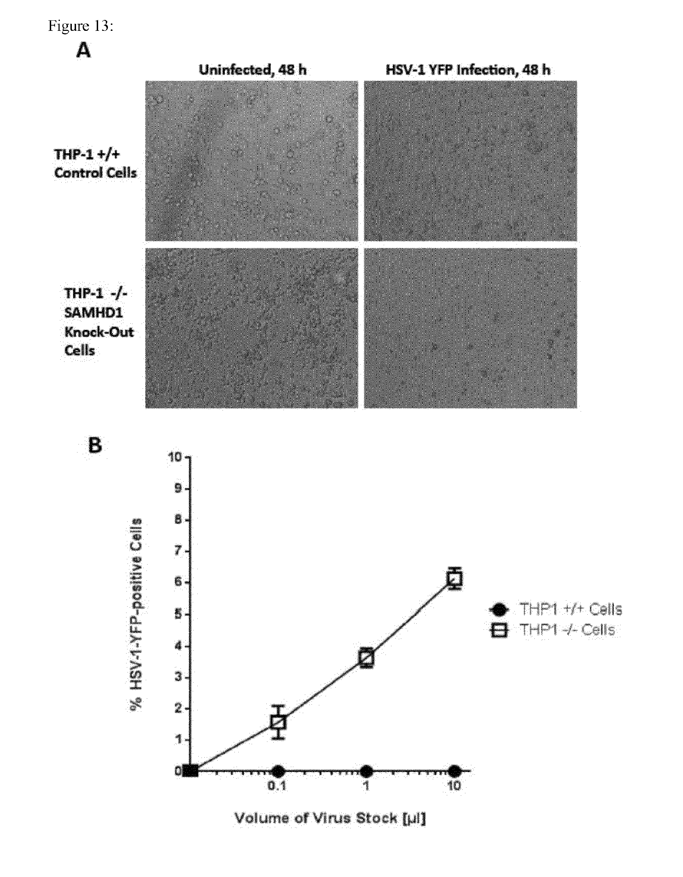

- **Figure 13:**: THP-1 (+/+) and THP-1 SAMHD1 (-/-) cells were challenged with different volumes of a HSV-1 YFP reporter virus and assessed for morphology and cell viability at time point 0 or at 48 h (uninfected and 10 µl HSV-1 YFP (**A**)) or assessed for the percentage of productively infected, YFP-positive cells 48 h post infection **(B).**

### EXAMPLES

### Materials and Methods

**Ethics statement.** Whole blood and bone marrow aspirates of AML patients were obtained and collected pre- and post-treatment. All patients gave informed consent according to the Declaration of Helsinki to participate in the collection of samples. The use of whole blood and bone marrow aspirates was approved by the Ethics Committee of Frankfurt University Hospital (approval no. SPO-01-2015).

**Cells and reagents.** Human leukemic cell lines from AML were obtained from DSMZ (Deutsche Sammlung von Mikroorganismen und Zellkulturen GmbH, Braunschweig, Germany) THP-1, OCI-AML2, OCI-AML3, Molm13 PL-21, HL-60, MV4-11, SIG-M5, ML2, NB4, KG1, MonoMac6, and HEL and cultured as previously reported⁷.

Ara-C resistant cell lines THP-1, HEL, HL-60, Molm13 and Molt4 were established by continuous exposure to increasing drug concentrations and are part of the Resistant Cancer Cell Line (RCCL) collection (http://www.kent.ac.uk/stms/cmp/RCCL/RCCLabout). The generation of resistant cell lines required 1 to 2 years, depending on the cell line used. All cell lines were maintained in IMDM supplemented with 10% FCS (SIG-M5 20% FCS), 200mM/ml L-Glutamine, 100 IU/ml penicillin, and 100 mg/ml streptomycin at 37°C in a humidified 5% CO₂ incubator. 293T cells were maintained as previously described ⁸.

THP-1 CRISP/Cas9 control and SAMHD1 knock-out cells were provided by Prof. Veit Hornung and cultivated in RPMI supplemented with 10% FCS, 100 IU/ml penicillin, and 100 ml/ml streptomycin. PDX cells were thawed and cultivated as previously described ⁹.

Peripheral blood mononuclear cells (PBMCs) from AML patients were purified by Ficoll-Hypaque gradient centrifugation ¹⁰. Leukemic cells were isolated from PBMCs by negative selection with a combination of CD3-, CD19- and CD235a-microbeads (Miltenyi Biotec) and separated by the autoMACS™ Pro Separator.

All primary cells were cultivated in X-vivo 10 medium (Lonza) supplemented with 10% Hy-Clone FCS (Perbio), 200mM/ml L-Glutamin, 25ng/ml hTPO, 50ng/ml hSCF, 50ng/ml hFlt3-Ligand and 20ng/ml hIL3 (all from Miltenyi Biotec) at 37°C in a humidified 5% CO₂ incubator.

The following fluorochrome-conjugated antibodies were used: CD33-PE, CD34-FITC (both from Miltenyi Biotech) and CD45-V450 (from BD Pharmingen). Additional antibodies were: rabbit anti-SAMHD1 (Proteintech), MHC-1 (W6-32 from Thermo Scientific), b-actin (BioVision via BioCat), dCK (Santa Cruz), phospho-SAMHD1 (T592P-antibody, generated by Eurogentec), Alexa-Fluor-488 and Alexa-Fluor-660 (both from Invitrogen, Life technologies).

**Plasmids.** The SIVmac251-based gag-pol expression constructs pSIV3+R- (Vpr-deficient) and pSIV3+X- (Vpx-deficient) were previously reported ¹¹. pLK0.1-puro-control-shRNA and pLK0.1-puro-SAMHD1-shRNA#1-3¹²¹ for shRNA-mediated silencing of SAMHD1 were previously described ¹². pHR-based transfer vectors expressing SAMHD1 wt and D137N, D207N, D311A, Q548A, T592A and T592D mutants were generated by site-directed mutagenesis in human codon-optimized SAMHD1 expression constructs (kindly provided by Dr. Thomas Gramberg) and subcloned into pHR-luc transfer vectors. pPAX2 was purchased from Addgene and pVSV-G has been previously described¹⁰.

**Cell viability assay.** Cell viability of the AML cell lines and primary AML cultures were determined by the 3-(4,5-dimethylthiazol-2-yl)-2,5-diphenyltetrazolium bromide (MTT) dye reduction assay after 96 hour incubation as described previously ⁷. Due to lower cell proliferation frequencies of the primary cells the CellTiter-Glo® Luminescent Cell Viability Assay (Promega) was used according to manufacturer's instruction. Here, the number of viable cells was determined 96 hours post treatment based on the quantification of the ATP levels.

**Immunoblotting.** Cells were lysed in Triton X-100 sample buffer and separated by sodium dodecyl sulfate-polyacrylamide gel electrophoresis. Proteins were blotted on a nitrocellulose membrane (Thermo Scientific), detected by specific antibodies: rabbit anti-SAMHD1 (Proteintech), b-actin (BioVision via BioCat), dCK (Santa Cruz), and phospho-SAMHD1 (T592P-antibody, generated by Eurogentec) and fluorescently labeled secondary antibodies (LI-COR) and visualized and quantified by infrared fluorescence protein detection (Odyssey LI-COR).

**dNTP measurement.** 5 x 10⁵ cells were treated with 10 µg/ml Ara-C 13C3 (Santa Cruz) and incubated at 37°C in a humidified 5% CO₂ incubator for 6 hours. Subsequently, cells were washed twice in 1x PBS, pelleted and stored at -20°C until measurement. The samples were analyzed at the Institute of Clinical Pharmacology (Goethe University Frankfurt) by liquid chromatography-electrospray ionization-tandem mass spectrometry as previously described ³. Briefly, the analytes were extracted by protein precipitation with methanol. An anion exchange HPLC column (BioBasic AX, Thermo) was used for the chromatographic separation and a 5500 QTrap (Sciex, Darmstadt, Germany) was used as analyzer, operating as triple quadrupole in positive multiple reaction monitoring (MRM) mode. Additional to the analytes reported in the publication, 13C3-Ara-CMP, 13C3-Ara-CDP and 13C3-Ara-CTP were quantified using the validated method and cytidine-¹³C₉,¹⁵N₃-5'-triphosphate as internal standard (IS). The precursor-to-product ion transitions used as quantifiers were *m*/*z* 327.0 → 115.1 for 13C3-Ara-CMP, *m*/*z* 407.0 → 115.1 for 13C3-Ara-CDP and *m*/*z* 487.0 → 115.1 for 13C3-Ara-CTP. A calibration curve was constructed for the quantitation of 13C3-Ara-CMP while no calibration standards were commercially available for 13C3-Ara-CDP and 13C3-Ara-CTP. For this reason, these analytes were quantified semiquantitatively by comparing the peak area ratios (analyte/IS) determined in the differently treated samples.

**Flow cytometry.** Detection of intracellular SAMHD1 was performed as previously described ¹. Additional stainings for surface markers (MHC-I, CD33, CD34, CD45) were applied prior to fixation. A FACSVerse or FACSCanto II flow cytometer (BD Biosciences) and FlowJo software (TreeStar) were used for analyses.

**RT-Q-PCR analyses.** RNA extraction and TaqMan-based mRNA quantification of SAMHD1 (Applied Biosystems: assay no. Hs00210019_m1) and RNaseP (Applied Biosystems: TaqMan® RNase P Control Reagents Kit (4316844), endogenous reference control) were performed in principle as reported ¹³.

miRNA extractions were performed according to manufacturer's instruction using Ambion PureLink® miRNA isolation kits (Thermo Fisher Scientific). cDNA conversions were performed as recommended using Applied Biosystems® TaqMan® MicroRNA Reverse Transcription kits and miRNA-specific primers. Following miRNAs were analyzed by quantitative PCR based on their significance for AML and predictive binding sites in the SAMHD1 genomic sequence based on targetscan.org, microcosm and microRNA.org: hsa-miRNA-181a (assay-ID: 000480); hsa-miRNA-181b (assay-ID: 001098); hsa-miRNA124a (assay-ID: 000446); hsa-miRNA150 (assay-ID: 000473); hsa-miRNA155 (assay-ID: 002623); hsa-miRNA30c (assay-ID: 000419); hsa-miRNA30d (assay-ID: 000420); hsa-miRNA142-5p (assay-ID:002248); RNU6B (reference control; assay-ID: 001093). Relative mRNA and miRNA levels were calculated as ΔCt and are presented relative to results obtained for THP-1 cells.

**Immunohistochemistry.** Paraffinized bone marrow aspirates were provided by the hematology/oncology department (University Hospital Frankfurt, Germany). SAMHD1 together with other hematopoietic markers was immunohistochemically stained and the expression of SAMHD1 in leukemic blasts was rated by board certified pathologist.

**Production of lentiviral expression vectors and VLPs**. Lentiviral vectors expressing SAMHD1 wt or SAMHD1 mutants were generated by co-transfection of packaging vector pPAX2, either pHR-SAMHD1 wt or pHR-mutant SAMHD1 and a plasmid encoding VSV.G. VLP stocks carrying either Vpx or Vpr from SIV_{mac251} were produced by co-transfection of 293T cells with pSIV3+ gag pol expression plasmids and a plasmid encoding VSV-G. The SAMHD1 degradation capacity of Vpx VLPs was determined on THP-1 cells 1 day post transduction by intracellular SAMHD1 staining.

**Manipulation of intracellular SAMHD1 levels.** For shRNA-mediated silencing of *SAMHD1,* OCI-AML3 cells were transduced by spinoculation with VSV-G pseudotyped lentiviral vectors carrying either pLK0.1-puro-control-shRNA or pLK0.1-puro-SAMHD1-shRNA#1-3¹². On day 30, transduced cells were cultivated in the presence of puromycin (7.5 µg/ml). Knockdown of SAMHD1 levels was monitored by intracellular SAMHD1 staining and Western blotting.

For re-constitution of SAMHD1, THP-1 CRISP/Cas9 SAMHD1 knock-out cells as well as HEL cells were transduced by spinoculation with VSV-G pseudotyped lentiviral vectors carrying SAMHD1 wt or mutants. Expression of SAMHD1 was monitored by intracellular SAMHD1 staining and Western blotting.

Cell lines and primary patient samples were transduced by spinoculation with VSV.G pseudotyped Vpx or Vpr VLPs. SAMHD1 levels were monitored by intracellular SAMHD1 staining. **HSV-1 YFP.** HSV-1 VP26-YFP virus (based on strain SC16, HSV-1 YFP) was produced on RITA cells as follows: a confluent monolayer of RITA cells was infected with HSV-1 YFP for 2-3 days, until a cytopathic effect was observed. After three freeze and thaw cycles, cellular debris was removed by centrifugation and virion-containing supernatants were collected and stored at -80°C.

### Example 1: A Positive Correlation Between SAMHD1 and Cytotoxicity of Cytarabine

SAMHD1 expression levels in 13 cell lines from AMLs were examined by Western Blot, flow cytometry and qPCR. Quantification of SAMHD1 protein levels by Western Blot or flow cytometry showed considerable differences in SAMHD1 expression in different cell lines (Fig. 1 and Fig. 2). Moreover, differences in SAMHD1 mRNA levels were noted (Fig. 2).

Next, it was explored whether SAMHD1 expression levels correlate with cytotoxic activity of cytarabine in different cell lines. As shown in Table 1, IC₅₀ values (concentrations inhibiting 50% of cell growth) of cytarabine ranged for AML cell lines from 7.8 to 560 ng/ml. Importantly, both protein and mRNA levels of SAMHD1 positively correlated with high statistical significance with IC₅₀ values of cytarabine (Fig. 3A). This suggested that the response of AML cells to cytarabine depends on SAMHD1 expression levels. In contrast, protein expression levels of dCK, a rate limiting enzyme in activation of cytarabine, did not correlate with IC₅₀ values of these cell lines (Fig. 1 and Fig. 3B).

### Example 2: Impairment of SAMHD1 Expression Increases Cytarabine Cytotoxicity while SAMHD1 Overexpression Reduces Cytarabine Cytotoxicity

To further elucidate the role of SAMHD1 in anti-leukemic activity of cytarabine, SAMHD1 was depleted in cell lines which express high endogenous levels of SAMHD1 (OCI-AML3, SIG-M5 and THP-1) using stable expression of SAMHD1 shRNA or disruption of the SAMHD1 gene using the CRISP/Cas9 technology. Expression of SAMHD1 shRNA in OCI-AML3 and SIG-M5 cells strongly reduced SAMHD1 protein levels, and in THP-1 CRISP/Cas9 knock-out cells SAMHD1 was undetectable (Fig. 4A). Importantly, SAMHD1 knock-down in OCI-AML-3 and SIG-M5 cells increased cytarabine toxicity by about 6-fold and 9-fold, respectively, compared to cells transduced with a control shRNA. In SAMHD1 knock-out THP-1 (THP-1 (-/-)) cells cytarabine toxicity increased by about 50-fold relative to the control THP-1 (THP-1(+/+)) cells (Fig. 4B). Additionally, it was tested whether transduction of wt SAMHD1 in HEL cell line, which express very low endogenous levels of SAMHD1, may reduce the sensitivity to cytarabine. As shown in Fig 4C,D, HEL cells over-expressing wt SAMHD1 after transduction were about 70-fold less sensitive to cytarabine when compared to the parental HEL cells or HEL cells expressing the enzymatically inactive SAMHD1 mutant D207N.

### Example 3: Cytarabine Triphosphate is a Direct Substrate of SAMHD1

SAMHD1 hydrolyzes intracellular dNTPs resulting in a reduction of intracellular dNTP pools. It was therefore tested whether SAMHD1 also affects cytarabine triphosphate levels in AML cells treated with cytarabine. To this end, THP-1 (-/-) and THP-1 (+/+) cells were incubated with 10 µg/ml cytarabine for 6 h, and cytarabine triphosphate was measured by liquid chromatography tandem mass spectrometry. About 20-fold higher cytarabine triphosphate amounts accumulated in THP-1(-/-) cells when compared with THP-1(+/+) cells suggesting that SAMHD1 decreases cytarabine triphosphate levels and, as a direct consequence, the anti-leukemic activity of cytarabine (Fig. 5A). An enzymatic *in vitro* assay with bacterially expressed SAMHD1 protein confirmed that cytarabine triphosphate serves as a direct substrate, but not as an activator, for SAMHD1's triphosphohydolase activity (Fig. 5B).

### Example 4: SAMHD1 Inhibitor Vpx-VLP Increases Cytarabine's Anti-Leukemic Activity

HIV-2 and some SIV strains encode for the accessory viral protein X (Vpx), which has the ability to target SAMHD1 to the proteasome for degradation. Virus-like particles pseudotyped with VSV-G that carry Vpx from SIV_{mac251} or other SIV and HIV-2 strains (Vpx-VLPs) were shown to reduce intracellular levels of SAMHD1. In macrophages and resting CD4 T-cells delivery or expression of Vpx resulted in a decrease of dNTP levels. Here, it was tested whether Vpx-VLPs may increase anti-leukemic activity of cytarabine through SAMHD1degradation in THP-1 cells. As shown in Fig. 6B, intracellular levels of SAMHD1 protein in THP-1 cells were reduced >10-fold 24 hours after treatment with Vpx-VLPs, while THP-1 cells treated with control VLP particles (Vpr-VLPs) showed similar SAMHD1 levels relative to the untreated cells. The strong reduction of SAMHD1 levels in Vpx-VLP-treated THP-1 cells persisted for more than 14 days. In addition, cytarabine's anti-leukemic activity increased in Vpx-VLPs treated THP-1 cells by about 20-fold (Fig. 6A). These results suggest that Vpx-VLPs possess therapeutic potential due to SAMHD1 degradation resulting in enhanced cytarabine toxicity in leukemic cells.

### Example 5: miRNA 181a/b Regulates SAMHD1 Expression in Leukemic Cells

Several microRNAs (miRNAs) were shown to target SAMHD1. For example, expression of miRNA181a was shown to decrease SAMHD1 expression in leukemic cells. Here, steady-state levels of miRNA181a and miRNA181b in different leukemic cell lines were tested. Both miRNAs were expressed to different extent in the panel of AML cell lines presented in Figs. 1 and 2 (Fig. 7). Moreover, the relative expression levels of miR181a and miR181b significantly correlated with expression levels of SAMHD1 in leukemic cell lines (p<0.05). These results suggest that miRNA181a/181b may be involved in the regulation of SAMHD1 und thus also the sensitivity to cytarabine in leukemic cells.

### Example 6: SAMHD1 Inhibition Overcomes Drug Resistance in AML Cells

AML patients frequently display at the start of therapy or develop resistance to therapy during treatment with cytarabine. Therefore, it was investigated whether AML cell lines with experimentally acquired resistance to cytarabine exert changes in SAMHD1 expression. For this aim, three AML cell lines, HEL, HL-60 and MOLM-13, with low endogenous SAMHD1 expression and high sensitivity to cytarabine were cultivated with increasing concentrations of cytarabine (up 2 µg/ml). Highly resistant cell sublines with IC₅₀ values of cytarabine ranging from 24 to 94 µg/ml were obtained. Remarkably, expression levels of SAMHD1 were increased in resistant cells relative to the parental cell lines (Fig. 8A and B). Enhanced expression of SAMHD1 in cytarabine resistant cell lines was accompanied with decreased cytarabine-triphosphate levels in resistant cells relative to the parental cell lines (Fig. 8C). Furthermore, reduction of SAMHD1 levels using Vpx-VLPs increased toxicity of cytarabine in all three cell lines tested (Fig. 8D).

In a next step, it was tested whether SAMHD1 affects anti-leukemic activity of other clinically relevant NAs. Cladribine, thiogunanine, and gemcitabine as well as topoisomerase II inhibitor daunorubicin inhibited proliferation of leukemic cells independently of SAMHD1 as demonstrated by similar IC₅₀ values in THP-1 (-/-) and THP-1 (+/+) cells (Fig. 9A). In contrast, IC₅₀ values of fludarabine, clofarabine and nelarabine were significantly lower in THP-1 (-/-) cells than in THP-1 (+/+) cells (5-fold for fludarabine; 5-fold for nelarabine and 2-fold for clofarabine) (Fig. 9A). Moreover, there was a significant correlation betweenIC₅₀ values of these NAs and SAMHD1 expression in different AML cell lines (Fig. 10). Together, these results and results obtained in HEL versus HEL SAMHD1 wt cells (Fig. 9B) suggest that anti-leukemic activity of Ara-G, fludarabine, clofarabine, nelarabine, and decitabine depends on expression levels of SAMHD1 albeit to a lesser extent than for cytarabine.

### Example 7: SAMHD1 Inhibition Enhances Anti-tumor Activity of Cytarabine in Glioma

Next, it was tested whether SAMHD1 may influence anti-tumoral activity of cytarabine in cultured cells from a malignant disease other than leukemia. As a solid tumor model we used glioma because SAMHD1 was previously shown to be expressed in glioma cells and cytarabine therapy was already tested in glioma patients. As shown Fig. 11, treatment of glioma cell line U251-MG with Vpx-VLPs resulted in an enhancement of the anti-tumoral activity of cytarabine by up to 10-fold.

### Example 8: SAMHD1-Expression Associates with Chemotherapy Responders

To determine the clinical relevance of SAMHD1 for AML therapy, a blinded SAMHD1 immunostaining of leukemic blasts in bone marrow samples of 72 AML patients taken prior to the start of standard cytarabine and daunorubicin-based combination therapy was performed by pathologists (examples shown in Fig. 12A). Here, the expression levels of SAMHD1 were evaluated in relation to a stratified therapy response: Patients classified as "responder" showed partial or complete AML remission, "non-responder" showed no clinical effect of combination therapy. This evaluation revealed statistically significant higher SAMHD1 expression in leukemic blasts of "non-responders" relative to "responders" (Fig. 12B).

### Example 9: SAMHD1 Inhibition Sensitizes Tumor Cells to Oncolytic Virus Treatment

To address whether SAMHD1 expression levels can modulate cancer cells' sensitivity to cytopathic and oncolytic effects of herpes viruses, THP-1 (+/+) and THP-1 SAMHD1 (-/-) cells were challenged with different volumes of an HSV-1 YFP reporter virus and assessed for morphology, cell viability and infection levels at different time points. THP-1 (-/-) cells lacking SAMHD1 were highly susceptible to HSV-1 infection, indicated by high percentages of productively infected cells (Fig. 13B), massive cytopathic effects and cell loss (Fig. 13A) 48 h post infection. In contrast, THP-1 (+/+) cells were nearly resistant to HSV-1 infection (Fig. 13B) and not killed by the oncolytic herpes virus (Fig. 13A). Thus, loss of SAMHD1 sensitized cancer cells to the cytopathic effect of herpes viruses.

### LITERATURE

1 Baldauf, H. M. et al. SAMHD1 restricts HIV-1 infection in resting CD4(+) T cells. Nature medicine 18, 1682-1687, doi:10.1038/nm.2964 (2012).
2 Pauls, E. et al. Cell cycle control and HIV-1 susceptibility are linked by CDK6-dependent CDK2 phosphorylation of SAMHD1 in myeloid and lymphoid cells. Journal of immunology 193, 1988-1997, doi:10.4049/jimmunol.1400873 (2014).
3 Thomas, D., Herold, N., Keppler, O. T., Geisslinger, G. & Ferreiros, N. Quantitation of endogenous nucleoside triphosphates and nucleosides in human cells by liquid chromatography tandem mass spectrometry. Analytical and bioanalytical chemistry 407, 3693-3704, doi:10.1007/s00216-015-8588-3 (2015).
4 Arnold, L. H., Kunzelmann, S., Webb, M. R. & Taylor, I. A. A continuous enzyme-coupled assay for triphosphohydrolase activity of HIV-1 restriction factor SAMHD1. Antimicrobial agents and chemotherapy 59, 186-192, doi:10.1128/AAC.03903-14 (2015).
5 Rowe, J. M. & Lowenberg, B. Gemtuzumab ozogamicin in acute myeloid leukemia: a remarkable saga about an active drug. Blood 121, 4838-4841, doi:10.1182/blood-2013-03-490482 (2013).
6 Raut, L. S. Novel formulation of cytarabine and daunorubicin: A new hope in AML treatment. South Asian journal of cancer 4, 38-40, doi:10.4103/2278-330x.149950 (2015).
7 Ogbomo, H., Michaelis, M., Klassert, D., Doerr, H. W. & Cinatl, J., Jr. Resistance to cytarabine induces the up-regulation of NKG2D ligands and enhances natural killer cell lysis of leukemic cells. Neoplasia 10, 1402-1410 (2008).
8 Keppler, O. T. et al. Rodent cells support key functions of the human immunodeficiency virus type 1 pathogenicity factor Nef. Journal of virology 79, 1655-1665, doi:10.1128/JVI.79.3.1655-1665.2005 (2005).
9 Vick, B. et al. An advanced preclinical mouse model for acute myeloid leukemia using patients' cells of various genetic subgroups and in vivo bioluminescence imaging. PloS one 10, e0120925, doi:10.1371/journal.pone.0120925 (2015).
10 Keppler, O. T. et al. Susceptibility of rat-derived cells to replication by human immunodeficiency virus type 1. Journal of virology 75, 8063-8073 (2001).
11 Gramberg, T., Sunseri, N. & Landau, N. R. Evidence for an activation domain at the amino terminus of simian immunodeficiency virus Vpx. Journal of virology 84, 1387-1396, doi:10.1128/JVI.01437-09 (2010).
12 Berger, A. et al. SAMHD1-deficient CD14+ cells from individuals with Aicardi-Goutieres syndrome are highly susceptible to HIV-1 infection. PLoS pathogens 7, e1002425, doi:10.1371/journal.ppat.1002425 (2011).
13 Goffinet, C., Schmidt, S., Kern, C., Oberbremer, L. & Keppler, O. T. Endogenous CD317/Tetherin limits replication of HIV-1 and murine leukemia virus in rodent cells and is resistant to antagonists from primate viruses. Journal of virology 84, 11374-11384, doi:10.1128/JVI.01067-10 (2010).

## Claims

1. An *in vitro* method for determining resistance of a cancer patient to a cancer therapy, comprising the steps of
a. Determining the level of SAM domain and HD domain-containing protein 1 (SAMHD1) in a sample from the cancer disease of the cancer patient,
b. Comparing the expression as determined in (a) with a control, or reference,
wherein the presence of, or higher level of, SAMHD1 in the sample compared to the control or reference indicates resistance of the cancer patient to the cancer therapy, or wherein the absence of, or lower level of, SAMHD1 in the sample compared to the control or reference indicates susceptibility of the cancer patient to the cancer therapy.

2. An *in-vitro* method for stratifying a cancer patient as responder or non-responder to a cancer therapy, comprising the steps of
a. Determining the level of SAMHD1 in a sample from the cancer disease of the cancer patient,
b. Comparing the expression as determined in (a) with a control or reference,
wherein the presence of, or higher level of, SAMHD1 in the sample compared to the control or reference indicates the cancer patient as non-responder to the cancer therapy, or wherein the absence of, or lower level of, SAMHD1 in the sample compared to the control or reference indicates the cancer patient as responder to the cancer therapy.

3. The method according to claim 1 or 2, wherein the cancer therapy is a chemotherapy, such as a therapy with a nucleoside analog (NA), or a therapy with an oncolytic virus, such as an oncolytic Herpes simplex virus (HSV).

4. An *in vitro* method of selecting a cancer therapy for treating a cancer patient, comprising the steps of
a. Determining the level of SAMHD1 in a sample from the cancer disease of the cancer patient,
b. Comparing the expression as determined in (a) with a control or reference,
wherein the absence of, or lower level of, SAMHD1 in the sample compared to the control or reference indicates a chemotherapeutic cancer therapy or a cancer therapy with an oncolytic virus for treating the cancer patient, and wherein the presence of, or a higher level of, SAMHD1 in the sample compared to the control or reference indicates a combination cancer therapy comprising a SAMHD1 inhibitor with a chemotherapeutic or oncolytic virus.

5. An *in vitro* method for monitoring a cancer therapy of a cancer disease, wherein during the cancer therapy, chemotherapy resistance of the cancer disease is determined according to an *in vitro* method of any of claims 1 to 3.

6. The method according to claim 5, wherein chemotherapy resistance of the cancer disease is determined at least twice during the chemotherapy, more preferably regularly during the therapy, e.g. monthly.

7. An NA or oncolytic virus for use in the treatment of a cancer disease in a subject, wherein the treatment comprises, obtaining from the subject a sample from the cancer disease, and determining chemotherapy resistance of the cancer disease in the subject according to a method of any of claims 1 to 3 using the sample from the cancer disease, wherein if a chemotherapy resistance is indicated for the cancer disease, the treatment is terminated, and wherein if no chemotherapy-resistance is indicated, the treatment is continued.

8. The NA or oncolytic virus of claim 7, wherein the NA is selected from didanosine, vidarabine, BCX4430, cytarabine, emtricitabine, lamivudine, zalcitabine, abacavir, aciclovir, entecavir, stavudine, telbivudine, zidovudine, idoxuridine, nelarabine, fludarabin, and trifluridine, and preferably is cytarabine; and wherein the oncolytic virus is an oncolytic HSV.

9. A SAMHD1 inhibitor for use in the treatment of a cancer disease, wherein the treatment comprises the concomitant or sequential administration of an SAMHD1 inhibitor and a second therapeutic selected from an NA or oncolytic virus.

10. The SAMHD1 inhibitor for use according to claim 9, wherein the cancer disease is a solid cancer disease, such as glioma, glioblastoma, or leukemia, and/or a recurrent cancer disease.

11. The SAMHD1 inhibitor for use according to claim 9 or 10, wherein SAMHD1 inhibitor is selected from a SAMHD1 inhibitory nucleic acid, such as an miRNA 181 a/b expression construct, an anti-SAMHD1 inhibitory antibody or T-cell receptor (TCR, inhibitory small molecule, or other SAMHD1 inhibitory proteins such as lentiviral protein X (Vpx) or viral protein R (Vpr), a molecule that acts as a negative regulator of SAMHD1 expression

12. The SAMHD1 inhibitor for use according to claim 11, wherein the accessory protein Vpx is encoded by HIV-2, SIV_{SM}, or SIV_{MAC}, and/or wherein the accessory protein Vpr is encoded by SIVₘᵤₛ and SIV_{deb}.

13. A combination of a SAMHD1 inhibitor and a second therapeutic selected from an oncolytic virus and NA, for use in the treatment of a cancer disease.

14. A pharmaceutical composition for use in a cancer disease comprising a SAMHD1 inhibitor and a second therapeutic selected from an oncolytic virus and NA.

15. A diagnostic kit, comprising means for determining the presence or absence of SAMHD1 in a sample from a cancer disease.
